Europäisches Patentamt

European Patent Office

Office européen des brevets

(11) Veröffentlichungsnummer: **0 298 227 B1**

(12) **EUROPÄISCHE PATENTSCHRIFT**

(45) Veröffentlichungstag der Patentschrift: **25.03.92**

(21) Anmeldenummer: **88108075.8**

(22) Anmeldetag: **20.05.88**

(51) Int. Cl.⁵: **B65B 3/02**, A61J 1/00

(54) Verfahren zum Befüllen und anschliessenden Verschweissen eines Behälters sowie Vorrichtung und Behälter zur Durchführung dieses Verfahrens.

(30) Priorität: **27.06.87 DE 3721308**

(43) Veröffentlichungstag der Anmeldung:
**11.01.89 Patentblatt 89/02**

(45) Bekanntmachung des Hinweises auf die
Patenterteilung:
**25.03.92 Patentblatt 92/13**

(84) Benannte Vertragsstaaten:
**CH DE ES FR GB IT LI**

(56) Entgegenhaltungen:
**CH-A- 502 229**
**FR-A- 2 213 873**
**FR-A- 2 293 298**
**GB-A- 1 059 969**

(73) Patentinhaber: **Hansen, Bernd**
**Heerstrasse 20**
**W-7166 Sulzbach-Laufen 2(DE)**

(72) Erfinder: **Hansen, Gerhard**
**Heerstrasse 20**
**W-7166 Sulzbach-Laufen 2(DE)**

(74) Vertreter: **Bartels, Hans et al**
**Patent Attorneys: Phys. H. Bartels Dipl.-Ing.**
**H. Fink Dr.-Ing. M. Held Lange Strasse 51**
**W-7000 Stuttgart 1(DE)**

## Beschreibung

Die Erfindung betrifft ein die Merkmale des Oberbegriffs des Anspruches 1 aufweisendes Verfahren zum Befüllen und anschließenden dichten Verschließen eines wenigstens in Teilbereichen elastisch verformbaren Behälters. Ferner betrifft die Erfindung eine Vorrichtung zum Durchführen dieses Verfahrens sowie einen nach dem Verfahren hergestellten Behälter.

Ein Verfahren dieser Art ist bereits aus der FR-A-2 213 873 bekannt. Obwohl bei dem bekannten Verfahren der Füllspiegel der eingefüllten Flüssigkeit im Behälter durch eine elastische Deformation desselben angehoben wird, bevor die Mündungsöffnung des Einfüllstutzens durch einen Schweißvorgang verschlossen wird, tritt bei dem bekannten Verfahren der Nachteil auf, daß die nach dem Verschweißen des Behälters in diesem noch enthaltende Restmenge an Luft verhältnismäßig groß ist. Handelt es sich bei dem Behälter um einen Beutel, der eine Infusionsflüssigkeit enthält, dann ist eine Druckinfusion, wie sie bei Unfällen erwünscht ist, ausgeschlossen, da die im Beutel enthaltene Luft durch den bei der Druckinfusion ausgeübten Druck in das Blut gelangen könnte. Bedingt ist dieses relativ große Restvolumen dadurch, daß der Füllspiegel sich in einem ausreichend großen Abstand von der Schweißstelle befinden muß, an welchem der Einfüllstutzen verschlossen wird.

Der Erfindung liegt daher die Aufgabe zugrunde, ein Verfahren der vorstehend genannten Art dahingehend zu verbessern, daß die im Behälter verbleibende Luftmenge wesentlich verringert werden kann. Diese Aufgabe löst ein Verfahren mit den Merkmalen des Anspruches 1.

Da durch die im ersten Schweißvorgang erfolgende Verkleinerung der Durchlaßöffnung des Einfüllstutzens dessen freibleibendes Volumen reduziert wird, genügt für die anschließende Anhebung des Füllspiegels eine relativ geringe Deformation des Behälters, um den größten Teil der zunächst im Behälter und im Einfüllstutzen noch vorhandenen Luft herauszudrücken. Wenn danach im zweiten Schweißvorgang die zuvor verbliebene Mündungsöffnung verschweißt wird, ist trotz des Abstandes, den der Füllspiegel von dieser Schweißstelle haben muß, das noch Luft enthaltende Restvolumen so gering, daß die darin befindliche Luft nicht mehr störend in Erscheinung tritt. Nach dem erfindungsgemäßen Verfahren mit einer Infusionsflüssigkeit gefüllte Beutel erlauben deshalb auch eine Druckinfusion.

Vorteilhafte Ausgestaltungen des Verfahrens gemäß Anspruch 1 sind Gegenstand der Unteransprüche 2 bis 4.

Der Erfindung liegt auch die Aufgabe zugrunde, eine Vorrichtung zur Durchführung des erfindungsgemäßen Verfahrens zu schaffen. Diese Aufgabe löst eine Vorrichtung mit den Merkmalen des Anspruches 5.

Bei dem Halter dieser Vorrichtung kann es sich um die Form handeln, in welcher ein aus Kunststoff bestehender Behälter im Blasverfahren hergestellt wird. Bekannte Formen dieser Art bedürfen nur insoweit einer Abwandlung, als sie eine Aussparung für die Verdrängungsvorrichtung benötigen, bei der es sich um einen Stempel handeln kann, mittels dessen vorübergehend die Wand des Behälters eingebeutelt wird.

Vorteilhafte Ausgestaltungen der Vorrichtung gemäß Anspruch 5 sind Gegenstand der Ansprüche 6 bis 8.

Ein nach dem erfindungsgemäßen Verfahren hergestellter, mit einer Flüssigkeit gefüllter und porendicht verschweißter Behälter weist vorzugsweise die Merkmale des Anspruches 9 auf.

Im folgenden ist die Erfindung anhand eines in der Zeichnung dargestellten Ausführungsbeispiels im einzelnen erläutert. Es zeigen:

Fig. 1    eine Seitenansicht des gefüllten und verschweißten Behälters,

Fig. 2    einen Schnitt durch den Behälter, die ihn aufnehmende Form und die Schweißvorrichtung.

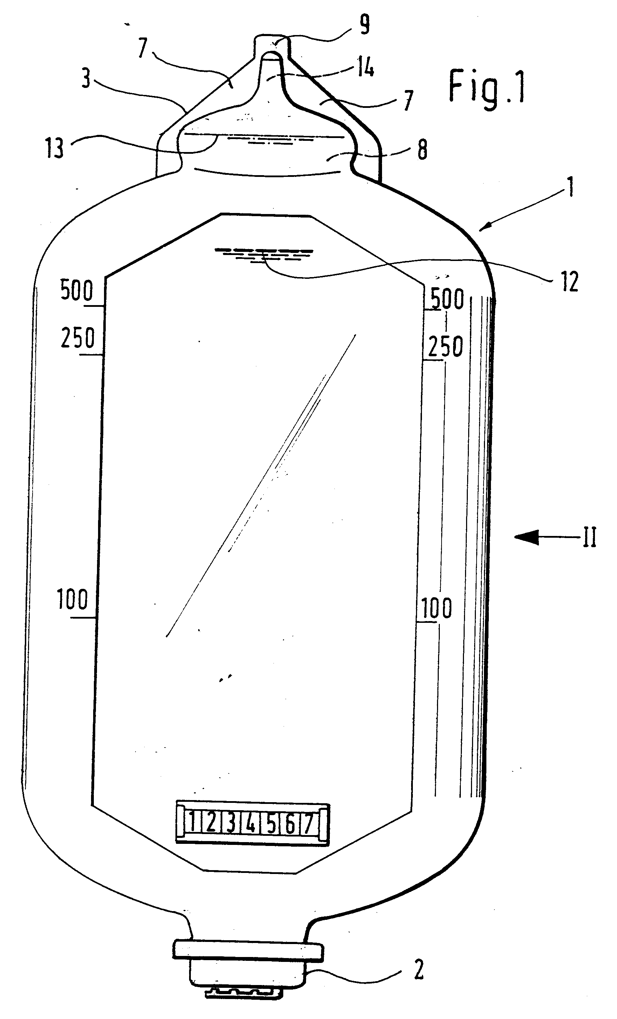

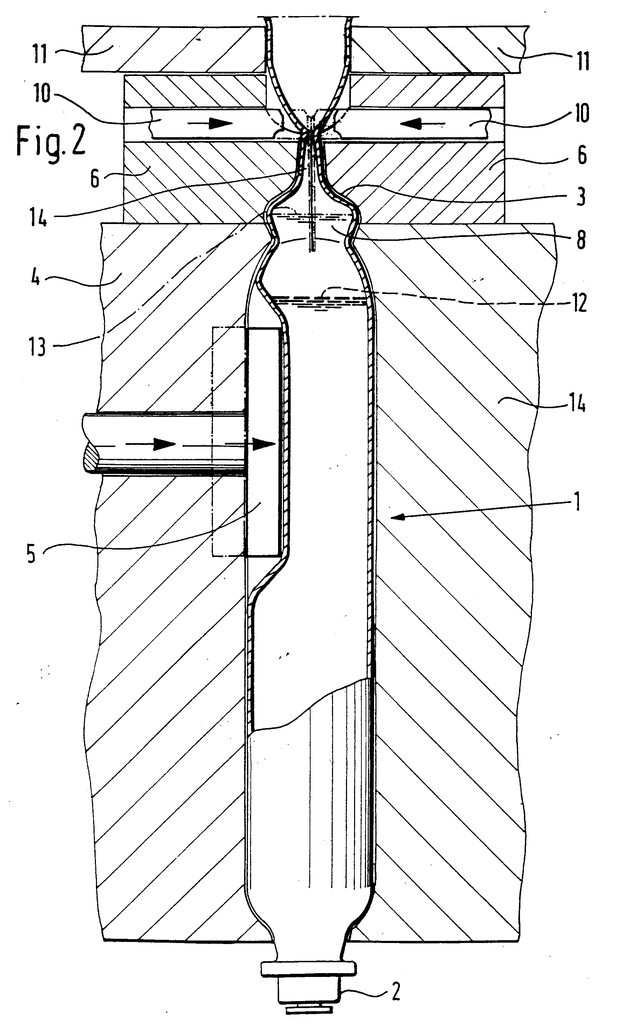

Ein aus Kunststoff im Blasverfahren hergestellter Beutel 1 zur Aufnahme einer Infusionsflüssigkeit hat, wie die Fig. 1 und 2 zeigen, die bei solchen Beuteln übliche, flache Form Sein beim Befüllen unten befindlicher Entnahmestutzen 2 ist in bekannter Weise ausgebildet. An seinem während des Befüllens nach oben weisenden Ende ist an den Beutel 1 ein Einfüllstutzen 3 angeformt, der zunächst eine vergrößerte Länge hat.

Die Herstellung des Beutels 1 erfolgt im Blasverfahren aus einem Schlauch, der in einem Formkörper 4 eingebracht wird, dessen Hohlraum eine mit der Form des Beutels 1 korrespondierende Form hat, wie Fig. 2 zeigt.

In dem die eine Seitenfläche des Beutels 1 bildenden Bereich ist der Formkörper 4 mit einer Aussparung versehen, in die ein Verdrängungsstempel 5 eingesetzt ist. Dieser Verdrängungsstempel 5 hat eine mit der Form der Aussparung übereinstimmende Kontur und liegt auf seinem ganzen Umfang zumindest im wesentlichen spielfrei an der Begrenzungswand der Aussparung an. Seine dem vom Formkörper 4 gebildeten Hohlraum zugekehrte Arbeitfläche schließt bündig mit der Innenfläche des Formkörpers 4 ab, wenn der Verdrängungsstempel 5 sich in seiner unwirksamen Stellung befindet. Er bildet dann einen Teil der Anlagefläche für den Beutel 1.

An der Oberseite des Formkörpers 4, über die der Einflußstutzen 3 übersteht, liegen zwei Kopfbacken 6 einer Schweißvorrichtung an, die gegeneinander bewegbar sind, um den Einflußstutzen 3 zu verschweißen. Die gegeneinander weisenden Arbeitsflächen dieser beiden Kopfbacken 6 drükken das den Einfüllstutzen 3 bildende Material nur längs ihres äußeren Randes aneinander, so daß nur hier eine porendichte Verschweißung erfolgt. Die beiden dabei erzeugten, verschweißten Randzonen sind in Fig. 1 mit 7 gekennzeichnet. Sie verlaufen, wie Fig. 1 zeigt, im Anschluß an den Beutel 1 zunächst parallel zueinander und nähern sich dann gleichmäßig bis auf einen Mindestabstand. Der nicht verschweißte Bereich 8 des Einfüllstutzens hat daher nach dem ersten Schweißvorgang die Form eines Trichters. In einem ersten, an den Innenraum des Beutels 1 anschließenden Abschnitt verengt sich die Durchlaßöffnung des Einfüllstutzens 3 stark. In dem sich anschließenden Endabschnitt, welcher dem Auslaufrohr des Trichters entspricht und der einen sehr kleinen Querschnitt hat, verengt sich die Durchlaßöffnung nur noch sehr geringfügig gegen die im verschweißten Zustand porendicht verschlossene Mündungsöffnung 9 hin.

Die Arbeitsflächen der Kopfbacken 6 enden ein wenig unterhalb der Mündungsöffnung 9. Hier sind die beiden Kopfbacken 6 mit je einer Ausnehmung versehen, in welcher zwei zusätzliche Backen 10 verschiebbar angeordnet sind. Die Verschieberichtung dieser zusätzlichen Backen 10 ist die gleiche wie diejenige der Kopfbacken 6, da sie die Aufgabe haben, die Mündungsöffnung 9 porendicht zu verschweißen. Ihre Arbeitsflächen sind deshalb so ausgebildet, daß zwischen ihnen das Material des Einflüllstutzens 3 vollflächig aneinander gedrückt wird. Die Backen 10 sind mit einer nicht dargestellten Heizvorrichtung beheizbar, damit auch dann, wenn bei einer schäumenden Flüssigkeit Schaum bis in die Höhe der Backen 10 gelangen sollte, eine porendichte Verschweißung erreicht wird.

Oberhalb ihrer Arbeitsflächen begrenzen die beiden Kopfbacken 6 einen sich nach oben erweiternden Raum, in dem sich ein Endabschnitt desjenigen Schlauches befindet, aus dem der Beutel 1 hergestellt worden ist. Oberhalb der Kopfbacken 6 angeordnete Haltebacken 11 halten diesen Endabschnitt des Schlauches in Anlage an ihren einander zugekehrten Arbeitsflächen, in welche Saugkanäle münden.

Nachdem der Behälter 1 im Formkörper 4 durch Blasen hergestellt und anschließend bis zu einer Höhe gefüllt worden ist, die in die Fig. 1 und 2 mit der Linie 12 gekennzeichnet ist und ein Stück weit unterhalb des unteren Endes des Einfüllstutzens 3 liegt, werden die beiden Kopfbacken 6 zusammengefahren. Dabei werden die Randzonen 7 miteinander verschweißt, wodurch der Einfüllstutzen 3 die aus den Fig. 1 und 2 ersichtliche, trichterartige Form erhält. Von der Durchgangsöffnung des Einfüllstutzens 3 ist dann nur noch der die Mündungsöffnung bildende Endabschnitt 14 offen.

Danach wird mittels des Verdrängungsstempels 5 die eine Seitenwand des Beutels 1 eingebeult. Dadurch wird der Flüssigkeitsspiegel von der Linie 12 bis etwa zu derjenigen Stelle innerhalb des Einfüllstutzens 3 angehoben, die in den Fig. 1 und 2 mit 13 gekennzeichnet ist. Diese Stelle liegt etwa dort, wo sich die Durchlaßöffnung des Einfüllstutzens zu verengen beginnt. Bei der Anhebung des Flüssigkeitsspiegels bis zur Linie 13 wird der größte Teil der zunächst im Beutel 1 und im Einfüllstutzen 3 noch vorhandenen Luft durch den Endabschnitt 14 hindurch nach außen verdrängt. Wenn nun der Endabschnitt 14 mit Hilfe der zusätzlichen Backen 10 porendicht verschweißt wird, ist der Abstand des Flüssigkeitsspiegels von dieser Schweißstelle noch genügend groß. Dennoch ist die Menge der sich noch im Einfüllstutzen 3 befindenden Luft vernachlässigbar gering, weil der Querschnitt der Durchlaßöffnung des Einfüllstutzens 3 durch den ersten Schweißvorgang erheblich reduziert worden ist.

Beim Verschweißen des Endabschnittes 14 wird mittels der zusätzlichen Backen 10 der überstehende Teil des Schlauches abgeschnitten. Außerdem wird im Anschluß an den zweiten Schweißvorgang der Verdrängungsstempel 5 wieder zurückgezogen, wodurch die Einbeulung des Beutels 1 wieder beseitigt wird. Der Beutel 1 kann deshalb in der in Fig. 1 dargestellten Form dem Formkörper 4 entnommen werden.

## Patentansprüche

1. Verfahren zum Befüllen und anschließenden dichten Verschließen eines wenigstens in Teilbereichen elastisch verformbaren Behälters, wobei an diesem ein Einfüllstutzen, dessen Durchlaßöffnung im Querschnitt kleiner ist als derjenige des Behälters, ausgebildet, letzterer über die Durchlaßöffnung des Einfüllstutzens mit Flüssigkeit gefüllt, der Füllspiegel der eingefüllten Flüssigkeit im Behälter durch eine elastische Deformation desselben in den Einfüllstutzen hinein angehoben und die Mündungsöffnung des Einfüllstutzens sodann durch einen Schweißvorgang porendicht verschlossen wird, dadurch gekennzeichnet, daß der Einfüllstutzen nach dem Befüllen durch einen ersten Schweißvorgang gebildet wird, bei dem der Einfüllstutzen zumindest in seinem Randbereich derart flachgedrückt und verschweißt wird, daß die Größe der Durchlaßöffnung des Einfüllstutzens verkleinert wird, daß sodann die Deformation des Behälters zum Anheben des Füllspiegels durchgeführt wird und daß danach das Verschließen der Mündungsöffnung als abschließender zweiter Schweißvorgang durchgeführt wird.

2. Verfahren nach Anspruch 1, dadurch **gekennzeichnet,** daß mit dem ersten Schweißvorgang dem die Mündungsöffnung bildenden Endabschnitt des Einfüllstutzens eine Querschnittsgröße gegeben wird, die gegenüber der Querschnittsgröße des zwischen diesem Endabschnitt und dem Behälterinnenraum liegenden Abschnitt wesentlich kleiner ist.

3. Verfahren nach Anspruch 2, dadurch **gekennzeichnet,** daß mit dem ersten Schweißvorgang der Durchlaßöffnung des Einfüllstutzens in dem an den Behälterinnenraum anschließenden Abschnitt eine sich gegen den Endabschnitt stark verengende und im Endabschnitt allenfalls nur noch wesentlich weniger verengende, insbesamt trichterähnliche Form gegeben wird.

4. Verfahren nach einem der Ansprüche 1 bis 3, dadurch **gekennzeichnet,** daß nach dem oder beim Verschließen des Einfüllstutzens der überstehende Teil des Einfüllstutzens vom verschlossenen Teil abgetrennt wird.

5. Vorrichtung zur Durchführung des Verfahrens gemäß Anspruch 1 mit einem den Behälter aufnehmenden Halter und einer Schweißvorrichtung mit einem an den Einfüllstutzen des Behälters anlegbaren, zusammenfahrbaren Backenpaar, dadurch **gekennzeichnet,** daß
    a) im Bereich des Halters (4) eine gegen den Behälter (1) bewegbare Verdrängungsvorrichtung (5) angeordnet ist,
    b) die Schweißvorrichtung zusätzlich zu dem ersten Backenpaar (6), dessen an den Einfüllstutzen (3) anlegbare Schweißflächen in Abstand von der Mündungsöffnung des Einfüllstutzens (3) enden, ein zweites Backenpaar (10) aufweist, dessen zusammenfahrbare Backen eine sich an die Schweißfläche des ersten Backenpaares (6) anschließende und bis zur Mündungsöffnung des Einfüllstutzens (3) erstreckende Schweißfläche haben.

6. Vorrichtung nach Anspruch 5, dadurch **gekennzeichnet,** daß die beiden Backen des ersten Backenpaares (6) durch je eine Kopfbacke gebildet sind, die mit je einer Führung für eine der beiden Backen des zweiten Backenpaares (10) versehen sind.

7. Vorrichtung nach Anspruch 5 und 6, dadurch **gekennzeichnet,** daß die Verdrängungsvorrichtung (5) durch einen in einem Wandteil des Halters (4) längsverschiebbar angeordneten Stempel gebildet ist.

8. Vorrichtung nach Anspruch 7, dadurch **gekennzeichnet,** daß der Halter (4) durch einen zweiteiligen Formkörper gebildet ist, dessen beide zusammenfahrbare Teile einen an die Form des Behälters (1) angepaßten Hohlraum begrenzen und dessen einer Teil mit einer Führung für den die Verdrängungsrichtung (5) bildenden Stempel versehen ist.

9. Nach dem Verfahren gemäß einem der Ansprüche 1 bis 4 hergestellter, mit einer Flüssigkeit gefüllter, zumindest in einem Teilbereich elastisch verformbarer Behälter, mit einem porendicht verschweißten Einfüllstutzen, dadurch **gekennzeichnet,** daß die Durchlaßöffnung des Einfüllstutzens (3) in dem an den Behälterinnenraum anschließenden Abschnitt eine gegen den sich an die verschlossene Mündungsöffnung anschließenden Endabschnitt (14) hin stark verengende und im anschließenden Endabschnitt eine sich allenfalls nur noch wesentlich weniger verengende, insgesamt trichterähnliche Form hat.

**Claims**

1. Method for filling and subsequently sealing a container which is elastically deformable at least in partial regions, a filling neck whereof the opening has a cross-section smaller than that of the container, being formed on the container, the container being filled with liquid through the opening in the filler neck, the filling level of the liquid introduced in the container being raised into the filler neck by an elastic deformation of the container and the opening of the filler neck then being sealed in a nonporous manner by a welding process, characterised in that after filling the filler neck is formed by a first welding process, during which the filler neck is pressed flat at least in its edge region and welded so that the size of the opening of the filler neck is reduced, that then the deformation of the container for raising the filling level is carried out and that subsequently the welding of the opening is carried out as a final second welding process.

2. Method according to Claim 1, characterised in that with the first welding process, the end section of the filler neck forming the opening is given a cross-sectional size, which is substantially smaller in comparison with the crosssectional size of the section located between this end section and the interior of the container.

3. Method according to Claim 2, characterised in that with the first welding process, in the section adjoining the interior of the container, the opening of the filler neck is given a shape narrowing considerably towards the end section and in the end section at best narrowing only substantially less, as a whole a funnel-like shape.

4. Method according to one of Claims 1 to 3, characterised in that after or during sealing of the filler neck, the projecting part of the filler neck is severed from the sealed part.

5. Apparatus for carrying out the method according to Claim 1 with a holder receiving the container and a welding device with a pair of jaws which can be brought together and can be applied to the filler neck of the container, characterised in that
    a) disposed in the region of the holder (4) is a displacement device (5) able to move towards the container (1),
    b) in addition to the first pair of jaws (6), whereof the welding surfaces able to be applied to the filler neck (3) terminate at a distance from the opening of the filler neck (3), the welding device comprises a second pair of jaws (10), whereof the jaws which can be moved together have a welding surface adjoining the welding surface of the first pair of jaws (6) and extending as far as the opening of the filler neck (3).

6. Device according to Claim 5, characterised in that the two jaws of the first pair of jaws (6) are respectively formed by a top jaw, which are respectively provided with a guide for one of the two jaws of the second pair of jaws (10).

7. Device according to Claim 5 and 6, characterised in that the displacement device (5) is formed by a ram arranged to move longitudinally in a wall part of the holder (4).

8. Device according to Claim 7, characterised in that the holder (4) is formed by a two-part shaped member, whereof both parts which can be moved together, define a cavity adapted to the shape of the container (1) and whereof one part is provided with a guide for the ram forming the displacement device (5).

9. Container produced according to the method according to one of Claims 1 to 4, filled with a liquid and elastically deformable at least in a partial region, with a filler neck welded in a non-porous manner, characterised in that in the section adjoining the interior of the container, the opening of the filler neck (3) has a shape narrowing considerably towards the end section (14) adjoining the sealed opening and in the adjoining end section a shape at best narrowing only substantially less, as a whole a funnel-like shape.

## Revendications

1. Procédé pour remplir et fermer ensuite hermétiquement un récipient dont certaines parties, au moins, sont élastiquement déformables, qui consiste à produire sur celui-ci un tube de remplissage (3) dont le canal a une section plus petite que celle du récipient, à remplir ce récipient avec un liquide par ledit canal, à élever le niveau du liquide remplissant le récipient jusqu'au tube de remplissage (3) en imposant audit récipient une déformation élastique avant de le fermer hermétiquement par une opération de soudage, caractérisé en ce qu'on forme le tube de remplissage (3), après le remplissage, par une première opération de soudage au cours de laquelle, au moins, la région du bord du tube de remplissage est aplatie et soudée, en ce qu'on diminue les dimensions du canal du tube de remplissage, en ce qu'on procède à la déformation du récipient jusqu'à ce que le niveau de remplissage de celui-ci a augmenté, et en ce que, après cela, on ferme l'orifice dudit tube de remplissage par une seconde opération de soudage.

2. Procédé selon la revendication 1, caractérisé en ce que, au cours de la première opération de soudage, produisant la partie d'extrémité constituant l'embouchure du tube de remplissage (9), on donne à celui-ci une section qui, comparativement à la section comprise entre cette partie d'extrémité (15) et l'espace intérieur du récipient, est sensiblement plus petite.

3. Procédé selon la revendication 2, caractérisé en ce que la première opération de soudage du canal du tube de remplissage, qui confère à la partie se raccordant à l'espace intérieur du récipient à une forme fortement rétrécissante à sa partie d'extrémité et ne rétrécit ensuite, tout au plus, que légèrement, lui donnant ainsi une forme générale en entonnoir.

4. Procédé selon l'une quelconque des revendications 1 à 3, caractérisé en ce que, après avoir fermé le tube de remplissage ou pendant, on détache la partie dépassante de celui-ci.

5. Dispositif pour la mise en oeuvre du procédé spécifié sous 1, comportant un support pour recevoir ledit récipient et un dispositif de soudage possédant deux mâchoires pouvant s'approcher l'une de l'autre et s'appliquer contre le tube de remplissage du récipient, caractérisé en ce que :

   a) à proximité du support (4) est monté un dispositif de refoulement ou de compression (5) pouvant venir s'appliquer contre le récipient (1),

   b) le dispositif de soudage comporte, en plus de la première paire de mâchoires (6), dont les surfaces de soudage, qui peuvent venir s'appliquer contre le tube de remplissage (3), se terminent à une certaine distance de l'orifice dudit tube de remplissage (3), une seconde paire de mâchoires (10), dont les mâchoires rapprochables possèdent une surface de soudage faisant suite à la surface de soudage de la première paire de mâchoires (6) et qui s'étend jusqu'à l'orifice du tube de remplissage (1).

6. Dispositif selon la revendication 5, caractérisé en ce que les deux mâchoires de la première paire de mâchoires (6) sont constituées, respectivement, par un élément de tête dont chacun est pourvu d'un guidage pour l'une des deux mâchoires de la seconde paire (10).

7. Dispositif selon les revendications 5 et 6, caractérisé en ce que le dispositif de refoulement (5) est constitué par un poussoir ou un piston pouvant coulisser longitudinalement dans une paroi du support (4).

8. Dispositif selon la revendication 7, caractérisé en ce que le support (4) se compose d'une pièce de forme en deux parties, dont les deux parties rapprochables délimitent un espace ou une chambre adaptée a la forme du récipient (1) et l'une des deux parties est pourvue d'un moyen pour guider le poussoir ou le piston constituant le dispositif de refoulement (5).

9. Récipient fabriqué selon l'une quelconque des revendications 1 à 4, rempli avec un liquide et dont une partie, au moins, est déformable élastiquement et qui comporte un tube de remplissage pouvant être fermé hermétiquement par soudage, caractérisé en ce que le canal du tube de remplissage (3) présente, dans sa partie se raccordant à l'espace intérieur du récipient, une forme rétrécissant fortement en direction de la section d'extrémité (14) qui se raccorde à l'embouchure fermée et dont la section d'extrémité qui suit a une forme rétrécissant nettement moins, conférant ainsi à l'ensemble une forme générale en entonnoir.

Fig.1

Fig.2